# EUROPEAN PATENT APPLICATION

(11) **EP 2 468 314 A1**
(43) Date of publication of application: **27.06.2012**
(21) Application number: 11191940.3
(22) Date of filing: 30.08.2007
(51) Int. Cl.: A61L 27/22, A61L 27/58, A61L 31/04, A61L 31/14

(54) **Medical devices having a coating for promoting endothelial cell adhesion**

(30) Priority: 06.09.2006 US 842384 P
(62) Divisional of application: 07837562.3
(71) Applicant: Boston Scientific Limited, Christ Church (BB)
(72) Inventor: Benco, John, Holliston, MA 01746 (US); Boden, Mark, Harris, RI 02830 (US); Brito, Shaina, Winchester, MA 01890 (US); Naimark, Wendy, Cambridge, MA 02138 (US); Pham, Lan, Nashua, NH (US)
(74) Representative: Vossius & Partner

(57) **Abstract**

A medical device having a coating of cell adhesion polypeptides to enhance endothelial cell adhesion onto the medical device. The cell adhesion polypeptides may be any of the proteins of the extracellular matrix which are known to play a role in cell adhesion or derivative peptides such as RGD or YIGSR. The polypeptides may be incorporated into the backbone of a polymer such as polyurethane, or grafted onto a polymer such polybisphosphonate. The polypeptides may also be carried on antibodies or displayed on bacteriophages. The polypeptides may also be modified to have adhesive amino acid sequences. In certain embodiments, the medical device further comprises a temporary barrier that protects the polypeptides from biofouling. The temporary barrier may be formed of a biodegradable polymer and be constructed as a coating over the polypeptides or as a plurality of micelles encapsulating the polypeptides. In certain embodiments, the polypeptides may be coated onto the medical device in such a manner as to form a monolayer of the polypeptides.

SEQUENCE LISTING

<110> BOSTON SCIENTIFIC LIMITED, et al.

<120> MEDICAL DEVICES HAVING A COATING FOR PROMOTING
      ENDOTHELIAL CELL ADHESION

<130> R1362 EP/2

<141> 2007-08-30

<150> 60/842,384
<151> 2006-09-06

<160> 2

<170> PatentIn Ver. 3.3

<210> 1
<211> 3
<212> PRT
<213> Artificial Sequence

<220>
<223> /note="Description of Artificial Sequence: Synthetic
      peptide"

<400> 1
Arg Gly Asp
  1

<210> 2
<211> 5
<212> PRT
<213> Artificial Sequence

<220>
<223> /note="Description of Artificial Sequence: Synthetic
      peptide"

<400> 2
Tyr Ile Gly Ser Arg
  1               5

## Description

### TECHNICAL FIELD

The present invention relates to implantable or insertable medical devices having bioactive coatings thereon.

### BACKGROUND

A problem associated with the use of vascular stents is reocclusion (restenosis) of the blood vessel after stent implantation. An important factor contributing to restenosis is the injury to or loss of the natural protective lining of endothelial cells on the inner surface of the artery as a result of stent implantation. This loss of the endothelial cell lining denudes the arterial wall, making it vulnerable to thrombosis, infection, scarring, or abnormal tissue growth. Thus, reestablishing a layer of endothelial cells (re-endothelialization) in the stented artery is thought to be important in improving the long-term biocompatibility of the stent. To promote effective endothelialization, however, endothelial cells must migrate from adjacent areas of the artery and adhere onto the surface of the stent.

It is known that certain proteins in the extracellular matrix, such as laminin, fibronectin, and collagen, are responsible for promoting endothelial cell adhesion. Additionally, various bioactive peptide sequences derived from these proteins, such as RGD and YIGSR, have been discovered to provide good substrates for endothelial cell adhesion.

Therefore, one approach to promoting re-endothelialization is by providing a surface coated with such bioactive peptides, such as the peptide-coated stent described in U.S. Patent Publication No. 2006/0052862 (Kanamaru et al.). Some have suggested that the peptides be incorporated into the backbone of polymers such as polyurethane, as described in U.S. Patent Publication No. 2006/0067909 (West et al.), which is incorporated by reference herein; or be grafted onto polymers, as described in Lin et al., Synthesis, Surface, and. Cell- Adhesion Properties of Polyurethanes Containing Covalently Grafted RGD-Peptides, J. Biomed. Materials Res. 28(3):329-42 (1994), which is incorporated by reference herein.

One of the problems associated with the use of such bioactive peptides *in vino* is biofouling of the peptides caused by the binding of plasma proteins or platelets onto the peptides. This biofouling defeats the ability of the peptides to bind to the target endothelial cells. One suggested approach to preventing biofouling is to incorporate the peptides into a hydrophilic polymer and grafting polyethylene glycol (PEG) onto the polymer. However, this method for protecting the peptides against biofouling has certain disadvantages. Thus, there is a need for an alternate method of preventing the biofouling of bioactive peptides. There is also a need for alternate methods of coating medical devices with bioactive peptides.

### SUMMARY OF THE INVENTION

The present invention provides a medical device at least partially coated with one or more cell adhesion polypeptides and a means for temporarily protecting the polypeptides from biofouling^{.} The cell adhesion polypeptides may be cell adhesion proteins of the extracellular matrix or peptides derived therefrom. The means for temporarily protecting may be a biodegradable barrier formed of a biodegradable polymer. The biodegradable barrier may be a coating at least partially disposed over the cell adhesion polypeptides or micelles encapsulating the polypeptides. The biodegradable barrier is designed to degrade in a time frame coincident with the process of re-endothelialization.

The present invention also provides a medical device having a coaling of cell adhesion polypeptides, wherein the polypeptides are grafted onto a polybisphosphonate.

The present invention also provides a medical device having a coating comprising bacteriophages, wherein the bacteriophages display cell adhesion polypeptides.

The present invention also provides a medical device having a coating of cell adhesion polypeptides, wherein the polypeptides are linked to adhesive polypeptide segments.

The present invention also provides a medical device having a coating comprising a monolayer of cell adhesion polypeptides.

The present invention also provides a method of providing a surface on a medical device for promoting endothelial cell adhesion onto the medical device, comprising the steps of coating at least a portion of the medical device with cell adhesion polypeptides, and applying a biodegradable polymer layer over at least a portion of the coating of cell adhesion polypeptides.

### BRIEF DESCRIPTION OF THE DRAWINGS

FIG. I shows a fragment of a polybisphosphonate with a cell adhesion peptide grafted thereon.

FIG. 2 is a schematic cross-section representation of a medical device according to the present invention with cell adhesion polypeptides carried on antibodies.

FIG. 3 is a schematic cross-section representation of a medical device according to the present invention with a coating of modified cell adhesion polypeptides.

FIG. 4 shows a fragment of a polybisphosphonate with a cell adhesion polypeptide-displaying bacteriophage grafted thereon.

### DETAILED DESCRIPTION

The present invention provides an implantable or insertable medical device having a coating of cell adhesion polypeptides to provide a substrate for the adhesion of endothelial cells onto the medical device. As used herein, the term "cell adhesion polypeptides" refers to compounds having at least two amino acids per molecule which are capable of binding endothelial cells via cell surface molecules, such as integrin, on endothelial cells. The cell adhesion polypeptides may be any of the proteins of the extracellular matrix which are known to play a role in cell adhesion, including fibronectin, vitronectin, laminin, elastin, fibrinogen, collagen types I, II, and V, as described in Boateng et al., RGD and YIGSR Synthetic Peptides Facilitate Cellular Adhesion Identical to That of Laminin and Fibronectin But Alter the Physiology of Neonatal Cardiac Myocytes, Am. J. Physiol. - Cell Physiol. 288:30-38 (2005), which is incorporated by reference herein. Additionally, the polypeptides may be any peptide derived from any of the aforementioned proteins, including fragments or sequences containing the binding domains. Such peptides include those having integrin-binding motifs, such as the RGD (arginine-glycine-aspartate) motif, the YIGSR (tyrosine-isoleucine-glycine-serine-arginine) motif, and related peptides that are functional equivalents. The peptides may also be any of the peptides described in U.S. Patent Publication No. 20060067909 (West et al.), which is incorporated by reference herein.

The cell adhesion polypeptides may be disposed on or within various types of surfaces on the medical device. In certain embodiments, the surface is the bare, uncoated surface of the medical device. The bare surface of the medical device may be smooth or porous, such as the porous stent surface described in U.S. Patent Publication No. 2005/0266040 (Gerberding), which is incorporated by reference herein. Where the surface is porous, the cell adhesion polypeptides may be deposited within the pores of the porous surface. In other embodiments, the surface of the medical device may be the surface of a coating on the medical device, such as a polymer coating. In any of the embodiments of the present invention, the polypeptides may be bonded to the surface of the medical device by any type of chemical or physical bonding means, including covalent, polar, ionic, coordinate, metallic, electrostatic, or intermolecular dipolar (including Van der Waals) bonds.

The cell adhesion polypeptides can be applied onto the surface of the medical device in various ways, including the use of coating methods that are known in the art. For example, the polypeptides may be sprayed onto the medical device by a conventional electrostatic spraying process, resulting in charged peptide-containing droplets being deposited onto the medical device. As the coating fluid dries, the polypeptides remain adhered to the medial device by inter-molecular bonding with the side-chain groups on the polypeptides. The deposited polypeptide may form a monolayer on the surface of the medical device, such as a Langmuir monolayer or a self-assembling monolayer as described in Van Alsten, Self-Assembled Monolayers on Engineering Metals: Structure, Derivation, and Utility, Langmuir 15:7605-14 (1999), which is incorporated by reference herein.

In certain embodiments, the cell adhesion polypeptides are incorporated into a polymer, which is then deposited onto a stent. Within certain embodiments, the polypeptides may be incorporated into the backbone of a polymer chain. For example, a polymer can be created containing YIGSR in the backbone of polyurethane as described in Jun et al., Development of a YIGSR-Peptide-Modified Polyurethaneurea to Enhance Endothelialization, J. Biomaterials Sci., Polymer Ed, 15(1):73-94 (2004), which is incorporated by reference herein. One of skill in the art could incorporate other cell adhesion polypeptides into the backbone of polyurethane or other polymers.

Within certain embodiments, the cell adhesion polypeptides may be grafted onto a polymer, which is then deposited onto the medical device. The polypeptides may be grafted onto a polymer using various methods known in the art. In one method, polymers having side branches containing reactive functional groups such as epoxide, halide, amine, alcohol, sulfonate, azido, anhydride, or carboxylic acid moieties can be covalently linked to the amine terminus of the polypeptides via the reactive side branches using conventional coupling techniques such as carbodiimide reactions. For example, RGD-containing peptides have been grafted onto the backbone of polyurethane, as described in Lin et al., Synthesis, Surface, and Cell-Adhesion Properties of Polyurethanes Containing Covalently Grafted RGD-Peptides, J. Biomedical Materials Res. 28(3):329-42 (1994). In another example, ROD-containing peptides have been grafted onto the side branches of polyethylene glycol based polymers, as described in Hansson et al., Whole Blood Coagulation on Protein Absorption-Resistant PEG and Peptide Functionalised PEG-Coated Titanium Surfaces, Biomaterials 26:861-872 (2005). One of ordinary skill in the art will also appreciate that polypeptides can be coupled to polymers via the carboxy-terminus of the polypeptides. For example, polymers with amine or hydroxyl side groups can be coupled to the carbnxy-terminus of polypeptides by carbodiimide or condensation reactions to create an amide or ester linkage.

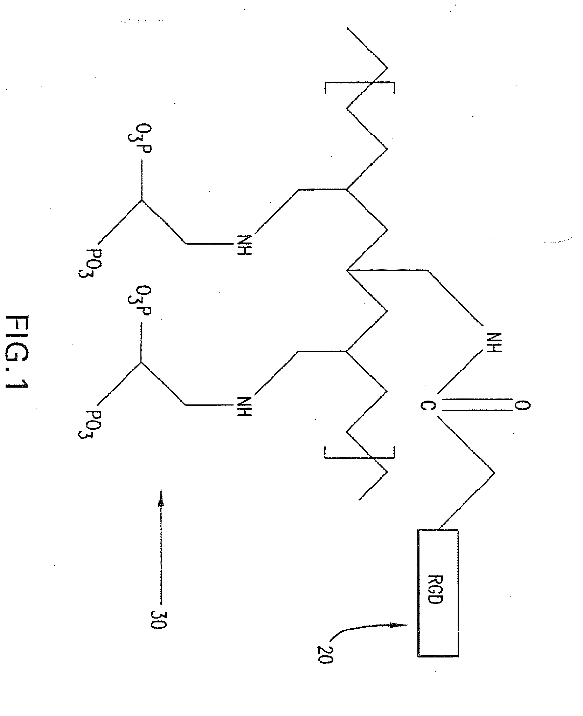

In another example, as shown in FIG. 1, the coating on a medical device may comprise cell adhesion polypeptides 20 (containing RGD in this particular examples) grafted onto polybisphosphonate 30. Polybisphosphonates that can be used to coat metallic substrates are described in Fishbein et al., Bisphosphonate-Mediated Gene Vector Delivery From the Metal Surfaces of Stents, Proc. Natl. Acad. Sci. 103(1):159-l64 (2006), which is incorporated by reference herein. Some polybispliosphonates, such as polyallylamine bisphosphonate, have amino functional groups which can be coupled to peptides via the carboxy-terminus using a carbodiimide coupling reaction. The polypeptide-grafted polybisphophonate may be coated onto the medical device as a monolayer, such as a Langmuir monolayer or a self-assembling monolayer. As used herein, a "self-assembled monolayer" refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array.

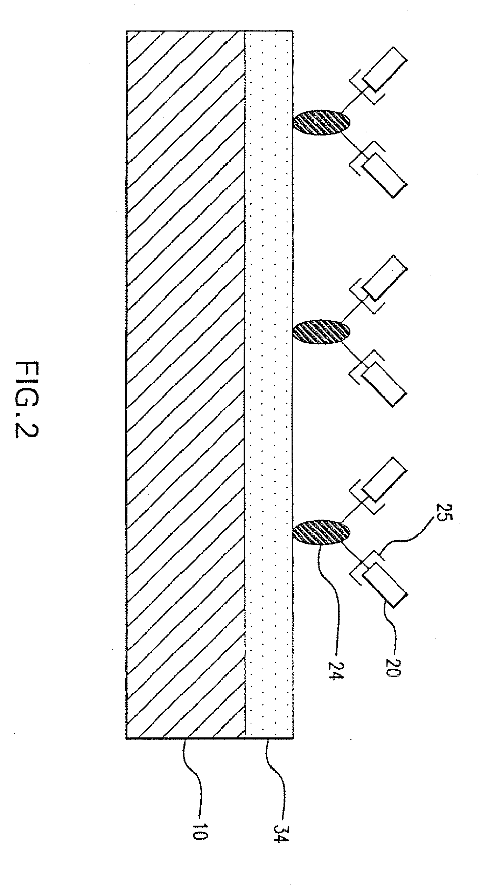

In certain embodiments, the coating on a medical device comprises cell adhesion polypeptides carried on antibodies. As used herein, the term "antibody" refers to an immunoglobulin, whether produced naturally or synthetically (e.g. recombinant), either in whole or in part. The term antibody also encompasses antibody fragments, which refers to any derivative of an antibody that is less than full length while retaining at least a portion of the full-length antibody's specific binding ability. Examples of antibody fragments include, but are not limited to, Fab, Fab', F(ab)₂, F(ab')₂, and Fv. As shown in FIG. 2, the antibodies 24 are conjugated to cell adhesion polypeptides 20 via the antigen binding site 25 of antibodies 24. The cell adhesion polypeptides may be conjugated to the antibodies prior to deployment of the medical device (e.g., during the manufacture of the medical device). Alternatively, it is also possible for the cell adhesion polypeptides to be conjugated to the antibodies after deployment of the medical device (e.g., by intravascular catheter delivery).

Antibodies 24 may be affixed onto a medical device 10 using various methods known in the art, including the method used to make the antibody-coated stents described in U.S. Patent Publication No. 2005/0043787 (Kutryk et al.), which is incorporated by reference herein. For example, medical device 10 may be coated with an antibody binding matrix 34 formed of synthetic materials (e.g., polyurethane, segmented polyurethane-urea/heparin, pokylactic acid, cellulose ester, or polyethylene glycol) or naturally occurring materials (e.g., collagen, laminin, heparin, fibrin, cellulose, or carbon). Antibodies 24 are tethered onto the matrix by either covalent or non-covalent bonding.

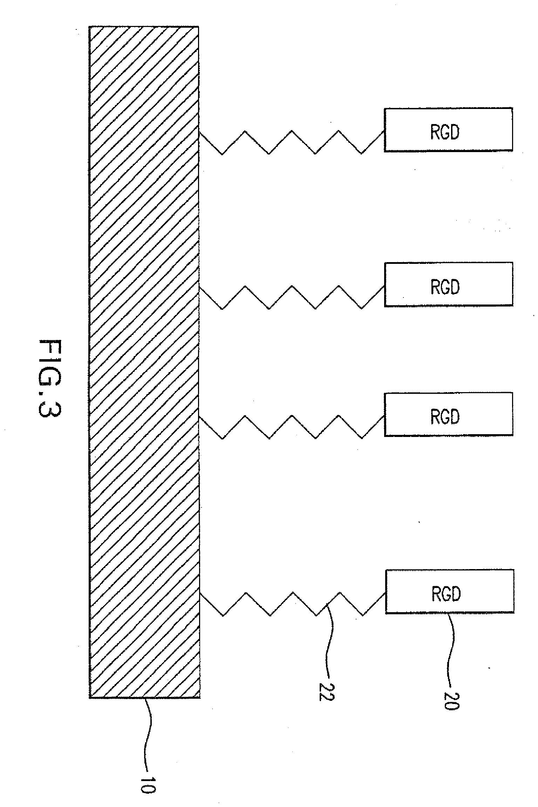

In certain embodiments, the cell adhesion polypeptides may be modified to enhance their adhesiveness to the surfaces of the medical devices. Peptides containing certain amino acids are known to have greater adhesion to inorganic surfaces, as described in Willet et al., Differential Adhesion of Amino Acids to Inorganic Surfaces, Proc. Natl. Acad. Sci. 102(22):7817-7822 (2005), which is incorporated by reference herein. The cell adhesion polypeptides used in the present invention may be modified to include such amino acids to promote adhesion to the surfaces of medical devices. For example, as shown in FIG. 3, a cell adhesion polypeptide 20 may be linked with an adhesive polypeptide segment 22 comprising a sequence of hydrophobic or charged amino acids, such as a polylysine tail. Adhesive polypeptide segment 22 is oriented towards the surface of medical device 10 so as to promote adhesion of polypeptide 20 onto medical device 10. The modified polypeptides may be coated onto the medical device as a monolayer, such as a Langmuir monolayer or a self-assembling monolayer.

In certain embodiments, the polypeptides may be displayed on a bacteriophage (phage). Phage display is the expression of polypeptides on the surface of bacteriophage particles. Phage display technology can be used to create pages for displaying a wide variety of polypeptides. *See* Willats, Phage Display: Practicalities and Prospects, Plant Molecular Bio. 50:837-854 (2002), which is incorporated by reference herein.

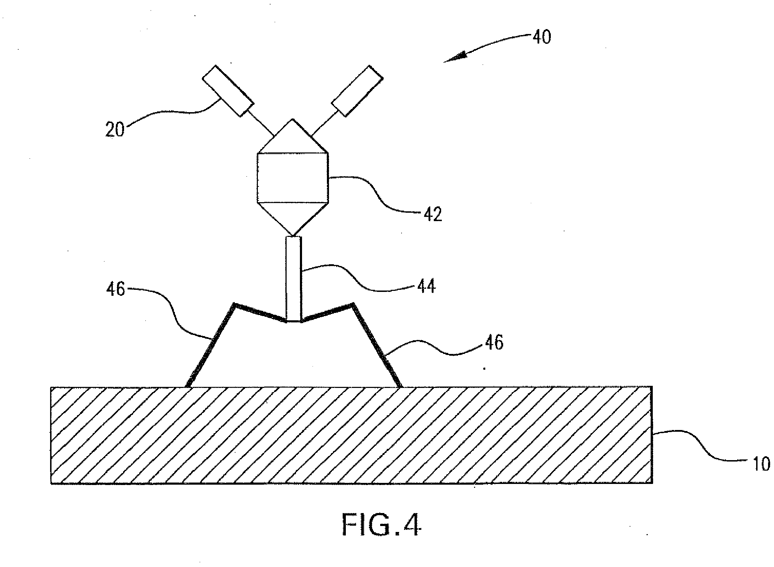

In this embodiment, as shown in FIG. 4, a bacteriophage 40 disposed on the surface of a medical device 10 has a head section 42, a tail section 44, and tail fibers 46. Head section 42 is modified to display polypeptides 20 on its surface. Further, tail fibers 46 are modified to include amino acids (e.g., positively charged amino acids) that would promote adherence to the surface of the medical device. For example, tail fibers 46 may be modified to include a polylysine sequence. Such modifications to the bacteriophage can be made through any conventional genetic engineering process, such as processes for altering the bacteriophage genes encoding for the proteins expressed in the head section and tail fibers.

Because endothelial cells must first migrate onto the stent surface before adhering to the coating of cell adhesion polypeptides, there is an interim period after implantation in which the cell adhesion polypeptide coating on the medical device is vulnerable to biofouling. Thus, in certain embodiments, the cell adhesion polypeptides are provided with a barrier means for temporarily protecting the polypeptides from biofouling. As used herein, the term "biofouling" refers to the binding of non-targeted materials, such as plasma proteins, platelets, and red blood cells, onto the polypeptides or the coating of polypeptides such that it interferes with the binding of targeted endothelial cells.

The temporary barrier is formed of a biodegradable material such as a biodegradable polymer and is designed to degrade upon implantation of the medical device and thereby expose the cell adhesion polypeptides to the physiologic environment in a timeframe coincident with the process of re-endothelialization. For a vascular stent, the process of re-endothelialization is known to begin very shortly after implantation. Time course analysis in rabbits has demonstrated almost 20% stent endothelialization 4 days after implantation and almost 40% after 7 days. *See* Belle et al., Stent Endothelialization: Time Course, Impact of Local Catheter Delivery, Feasibility of Recombinant Protein Administration, and Response to Cytokine Expedition, Circulation 95:438-448 (1997), which is incorporated by reference herein. As well known in the art, the biodegradation rate of a biodegradable polymeric barrier may be controlled by various factors such as the composition, structure, and thickness of the barrier. Therefore, one of ordinary skill in the art can design a biodegradable barrier to degrade and expose the underlying cell adhesion polypeptides in a timeframe coincident with the process of re-endothelialization, while minimizing the opportunity for biofouling of the polypeptides. For example, the biodegradable barrier may be designed to degrade such that the polypeptides are exposed within 4 days or within 7 days after implantation. Certain polymers of poly(L-lactide-co-glycolide) and poly(L-lactide) having various degradation rates are reported in Zilberman et al., Dexamethasone loaded bioresorbable films used in medical support devices: Structure, degradation, crystallinity and drug release, Acta Biomaterialia 1:615-624 (2005), which is incorporated by reference herein. This polymer degradation rate has been shown to be adjustable by varying the lactide to glycolide ratio as well as by varying the chiral configuration of the lactide monomer. In addition, the thickness of the coating can be adjusted to fine tune the rate at which the polypeptides are exposed, yielding a wide range of possible exposure profiles.

Within certain embodiments, the temporary barrier is a biodegradable polymer coating at least partially disposed over the cell adhesion polypeptides. The polypeptides may be disposed on the surface of the medical device by using any method known in the art, including conventional coating techniques such as spray coating, electrostatic spray coating, and dip coating. The polypeptide may also be disposed on the surface of the medical device by any of the methods disclosed in the aforementioned embodiments. The biodegradable coating may be applied onto the medial device using any known method of applying such coatings to the surfaces of medical devices.

Examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L-lactide), poly(lactic acid-co-glycolic acid), 50/50 (DL-lactide-co-glycotide); polydioxanone; polypropylene fumarate; polydepsipeptides; polycaprolactone and co-polymers and mixtures thereof such as poly(D,L-lactide-co-caprolactone) and polycaprolactone co-butylarrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

In certain embodiments, the temporary barrier comprises a plurality of biodegradable vesicles, wherein the cell adhesion polypeptides are encapsulated within the vesicles. The vesicles may be micelles, liposomes, lipospheres, microspheres, microbubbles, and the like, and may be formed of polymers or lipids. As described above, the vesicle walls can be designed to degrade in a time frame coincident with the process of re-endothelialization. Degradation of the vesicles will release the polypeptides, which can then precipitate onto the medial device surface and adhere thereto. The vesicles may be disposed on the medical device in various ways known in the art. For example, the vesicles may be embedded within a porous surface on the medical device.

Within certain embodiments, the medical device may further comprise therapeutic agents. The therapeutic agent may be carried on or within any component of the medical device, including on or within the temporary protective barrier or another polymer coating on the medical device. In some instances, the therapeutic agent may be provided on a surface of the medical device by any of the methods by which the cell adhesion polypeptides are adhered thereto. In fact, the therapeutic agent may be provided on the same surface as the cell adhesion polypeptides.

The therapeutic agents may be agents that promote angiogenesis or the activation, recruitment, or migration of endothelial cells. For example, angiogenic factors such as PD-ECGF (platelet-derived endothelial cell growth factor) or VEGF (vascular endothelial growth factor), or endothelial cell chemoattractants such as 2-deoxy-D-ribose could be released from the medical device to recruit endothelial cells onto the medical device.

The therapeutic agent may also be any pharmaceutically acceptable agent such as a non-genetic therapeutic agent, a biomolecule, a small molecule, or cells.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); anti-proliferative agents such as enoxaparin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl) ethyleneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; protein-bound particle drugs such as ABRAXANE™; and any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMP's"), such as, for example, BMP-2, BMP-3, BMP-4, BMP-5, BMP-6 (VGR-1), BMP-7 (OP-1), BMP-8, BMP-9, BMP-10, BMP-11, BMP-12, BMP-13, BMP-14, BMP-15. Preferred BMP's are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factors α and β, platelet-derived endothelial growth factor, platelet-derived growth factor, tumor necrosis factor a, hepatocyte growth factor, and insulin-like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase and combinations thereof and other agents useful for interfering with cell proliferation.

Exemplary small molecules include hormones, nucleotides, amino acids, sugars, and lipids and compounds have a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin-) cells (including Lin-CD34⁻, Lin⁻CD34⁺, Lin⁻cKit ⁺, mesenchymal stem cells including mesenchymal stem cells with 5-aza, cord blood cells, cardiac or other tissue derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells. Any of the therapeutic agents may be combined to the extent such combination is biologically compatible.

Non-timiting examples of medical devices that can be used with the present invention include stents, stent grafts, catheters, guide wires, neurovascular aneurysm coils, balloons, filters (*e.g.,* vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like.

The foregoing description and examples have been set forth merely to illustrate the invention and are not intended to be limiting. Each of the disclosed aspects and embodiments of the present invention may be considered individually or in combination with other aspects, embodiments, and variations of the invention. In addition, unless otherwise specified, none of the steps of the methods of the present invention are confined to any particular order of performance. Modifications of the disclosed embodiments incorporating the spirit and substance of the invention may occur to persons skilled in the art and such modifications are within the scope of the present invention. Furthermore, all references cited herein are incorporated by reference in their entirety.
The invention is summarized by the following items:
1. A medical device having a coating over at least a portion of the medical device, wherein the coating comprises:
   (a) one or more cell adhesion polypeptides; and
   (b) a means for temporarily protecting the polypeptides from biofouling.
2. The medical device of item 1, wherein the cell adhesion polypeptides are peptides derived from a binding domain of a cell adhesion protein of the extracellular matrix.
3. The medical device of item 2, wherein the cell adhesion protein is selected from the group consisting of fibronectin, vitronectin, laminin, elastin, fibrinogen, collagen type 1, collagen type 11, and collagen type V.
4. The medical device of item 2, wherein the peptides comprise an amino acid sequence selected from the group consisting of arginine-glycine-aspartate (RGD) and tyrosine-isoleucine-glycine-serine-arginine (YIGSR).
5. The medical device of item 1, wherein the cell adhesion polypeptides form a monolayer on the surface of the medical device.
6. The medical device of item I, wherein the means for temporarily protecting comprises a biodegradable barrier which is at least partially disposed over or surrounds the polypeptides.
7. The medical device of item 6, wherein the biodegradable barrier comprises a biodegradable polymer.
8. The medical device of item 7, wherein the biodegradable barrier comprises biodegradable micelles, and wherein the polypeptides are encapsulated within the micelles.
9. The medical device of item 7, wherein the biodegradable barrier comprises a biodegradable coating, and wherein the biodegradable coating is at least partially disposed over the polypeptides.
10. The medical device of item 7, wherein the degradation of the biodegradable barrier upon implantation of the medical device coincides with the process of re-endothelialization of the medical device.
11. The medical device of item 7, wherein the biodegradable barrier degrades at a rate such that the cell adhesion polypeptides are at least partially exposed to a physiologic environment within 7 days after implantation of the medical device in a patient.
12. The medial device of item 11, wherein the biodegradable barrier degrades at a rate such that the cell adhesion polypeptides are substantially exposed to a physiologic environment within 7 days after implantation of the medical device in a patient.
13. The medical device of item 11, wherein the biodegradable barrier substantially degrades within 7 days after implantation of the medical device in a patient.
14. The medical device of item 7, wherein the biodegradable barrier degrades at a rate such that the cell adhesion polypeptides are at least partially exposed to a physiologic environment within 4 days after implantation of the medical device in a patient.
15. The medical device of item 14, wherein the biodegradable barrier degrades at a rate such that the cell adhesion polypeptides are substantially exposed to a physiologic environment within 4 days after implantation of the medical device in a patient.
16. The medical device of item 14, wherein the biodegradable barrier substantially degrades within 4 days after implantation of the medical device in a patient.
17. A medical device having a coating comprising cell adhesion polypeptides, wherein the cell adhesion polypeptides are grafted onto a polybisphosphonate.
18. The medical device of item 17, wherein the polybisphosphonate is polyallylamine bisphosphonate.
19. The medical device of item 17, wherein the cell adhesion polypeptides form a monolayer on the surface of the medical device.
20. The medical device of item 17, further comprising a means for temporarily protecting the polypeptides from biofouling.
21. A medical device having a coating comprising a bacteriophage, wherein the bacteriophage displays cell adhesion polypeptides.
22. The medical device of item 21, wherein the medical device further comprises a polybisphosphonate coating, and wherein the bacteriophage is grafted onto the polybisphosphonate coating.
23. The medical device of item 22, wherein the polybisphosphonate is polyallylamine bisphosphonate.
24. The medical device of item 21, further comprising a means for temporarily protecting the polypeptides from biofouling.
25. A medical device having a coating comprising cell adhesion polypeptides, wherein the polypeptides are linked to adhesive polypeptide segments.
26. The medical device of item 25, wherein the adhesive polypeptide segment is polylysine.
27. The medical device of item 25, wherein the adhesive polypeptide segment is a chain of hydrophobic amino acids.
28. The medical device of item 25, wherein the cell adhesion polypeptides form a monolayer on the surface of the medical device.
29. The medical device of item 25, further comprising a means for temporarily protecting the polypeptides from biofouling.
30. A medical device having a coating comprising a monolayer of cell adhesion polypeptides.
31. The medical device of item 30, wherein the cell adhesion polypeptides form a monolayer directly on the surface of the medical device.
32. The medical device of item 30, wherein the monolayer is a self-assembled monolayer.
33. The medical device of item 30, wherein the monolayer is a Langmuir monolayer.
34. The medical device of item 30, wherein the cell adhesion polypeptides are applied onto the medical device by electrostatic spraying.
35. The medical device of item 30, wherein the cell adhesion polypeptides are linked to adhesive polypeptide segments.
36. The medical device of item 30, wherein the cell adhesion polypeptides are grafted onto a polymer.
37. The medical device of item 30, wherein the cell adhesion polypeptides are incorporated into the backbone of a polymer.
38. The medical device of item 30, further comprising a means for temporarily protecting the polypeptides from biofouling.
39. A method of providing a surface on a medical device for promoting endothelial cell adhesion onto the medical device, comprising the steps of:
   (a) coating at least a portion of the medical device with cell adhesion polypeptides; and
   (b) applying a biodegradable polymer layer over at least a portion of the coating of cell adhesion polypeptides.
40. The method of item 39, wherein the degradation of the biodegradable polymer layer upon implantation of the medical device coincides with the process of re-endothelialization of the medical device.

## Claims

1. A medical device having a coating comprising cell adhesion polypeptides, wherein the polypeptides are linked to adhesive polypeptide segments.

2. The medical device of claim 1, wherein the adhesive polypeptide segment is polylysine.

3. The medical device of claim 1, wherein the adhesive polypeptide segment is a chain of hydrophobic amino acids.

4. The medical device of claim 1, wherein the cell adhesion polypeptides form a monolayer on the surface of the medical device.

5. The medical device of claim 1, further comprising a means for temporarily protecting the polypeptides from biofouling.

6. The medical device of claim 5, wherein the means for temporarily protecting comprises a biodegradable barrier which is at least partially disposed over or surrounds the polypeptides.

7. The medical device of claim 6, wherein the biodegradable barrier comprises a biodegradable polymer.

8. The medical device of claim 1, wherein the cell adhesion polypeptides are peptides derived from a binding domain of a cell adhesion protein of the extracellular matrix.

9. The medical device of claim 8, wherein the cell adhesion protein is selected from the group consisting of fibronectin, vitronectin, laminin, elastin, fibrinogen, collagen type I, collagen type II, and collagen type V.

10. The medical device of claim 8, wherein the peptides comprise an amino acid sequence selected from the group consisting of arginine-glycine-aspartate (RGD) and tyrosine-isoleucine-glycine-serine-arginine (YIGSR).

11. The medical device of claim 1, wherein the cell adhesion polypeptides form a monolayer on the surface of the medical device.
